# EUROPÄISCHE PATENTSCHRIFT

(11) **EP 4 190 856 B1**
(45) Veröffentlichungstag und Bekanntmachung des Hinweises auf die Patenterteilung: **01.04.2026**
(21) Anmeldenummer: 22205081.7
(22) Anmeldetag: 02.11.2022
(51) Int. Cl.: C08L 7/00

(54) **VERBINDUNG, KAUTSCHUKMISCHUNG ENTHALTEND DIE VERBINDUNG, FAHRZEUGREIFEN, DER DIE KAUTSCHUKMISCHUNG IN WENIGSTENS EINEM BAUTEIL AUFWEIST, VERFAHREN ZUR HERSTELLUNG DER VERBINDUNG SOWIE VERWENDUNG DER VERBINDUNG ALS ALTERUNGSSCHUTZMITTEL UND/ODER ANTIOXIDATIONSMITTEL UND/ODER FARBSTOFF**
COMPOUND, RUBBER COMPOUND CONTAINING THE COMPOUND, VEHICLE TIRE CONTAINING THE RUBBER COMPOUND IN AT LEAST ONE COMPONENT, METHOD FOR PRODUCING THE COMPOUND, AND USE OF THE COMPOUND AS AN ANTI-AGING-AGENT AND/OR ANTIOXIDANT AND/OR COLORANT
COMPOSÉ, MÉLANGE DE CAOUTCHOUC CONTENANT LE COMPOSÉ, PNEU DE VÉHICULE COMPRENANT LE MÉLANGE DE CAOUTCHOUC DANS AU MOINS UN COMPOSANT, PROCÉDÉ DE FABRICATION DU COMPOSÉ ET UTILISATION DU COMPOSÉ COMME AGENT ANTI-VIEILLISSEMENT ET/OU ANTIOXYDANT ET/OU COLORANT

(30) Priorität: 02.12.2021 DE 102021213721
(43) Veröffentlichungstag der Anmeldung: 07.06.2023
(73) Patentinhaber: Continental Reifen Deutschland GmbH, 30175 Hannover (DE)
(72) Erfinder: Jacob, Andreas, 30165 Hannover (DE); Dauer, David-Raphael, 30165 Hannover (DE); Strohmeier, Julian, 30165 Hannover (DE); Becker, Jörg-August, 30167 Hannover (DE); Matz, Florian, 30167 Hannover (DE)
(74) Vertreter: Continental Corporation

(56) Entgegenhaltungen:
- EP-A1- 1 694 318
- EP-A1- 3 517 570
- US-A- 3 389 124

## Beschreibung

Die Erfindung betrifft eine Verbindung, eine Kautschukmischung enthaltend die Verbindung, einen Fahrzeugreifen, der die Kautschukmischung in wenigstens einem Bauteil aufweist, Verfahren zur Herstellung der Verbindung sowie die Verwendung der Verbindung als Alterungsschutzmittel und/oder Antioxidationsmittel und/oder Farbstoff.

Es ist bekannt, dass in Fahrzeugreifen und technischen Gummiartikeln polymere Materialien, wie insbesondere Kautschuke, eingesetzt werden.

Naturkautschuk, synthetische Polymere (wie IR, BR, SSBR, ESBR, etc.) aber auch natürliche sowie synthetische Öle, Fette und Schmiermittel unterliegen bei längerer Lagerung und vor allem in der Zielanwendung, die oft bei höheren Temperaturen abläuft, Oxidationsreaktionen, die sich nachteilig auf die ursprünglichen gewünschten Eigenschaften auswirken. Je nach Art des Polymers werden die Polymerketten, bis hin zu einer Verflüssigung des Materials verkürzt oder es kommt zur nachträglichen Härtung des Werkstoffes.

US 3,389,124 offenbart beispielsweise Amino-substituierte Phenoxazine für Kautschukmischungen, um diese vor oxidativem Abbau zu schützen.

Alterungsschutzmittel tragen daher maßgeblich zur Langlebigkeit von Fahrzeugreifen und anderen technischen Gummiartikeln bei.

Bekannte Alterungsschutzmittel sind aromatische Amine, wie beispielsweise 6PPD (N-(1,3-Dimethylbutyl)-N'-phenyl-p-phenylendiamin), I PPD (N-Isopropyl-N'-phenyl-p-phenylendiamin) oder SPPD (N-(1-phenylethyl)-N'-phenyl-p-phenylendiamin).

Diese Moleküle können mit Sauerstoff oder Ozon oder gebildeten Radikalen, wie Alkyl-, Alkoxy- und Alkylperoxyradikalen, reagieren und diese somit abfangen und somit die Kautschuke etc. vor weiteren Oxidationsreaktionen schützen.

Nachteilig an dieser Substanzklasse ist allerdings der Verdacht, dass diese krebserregend sein könnten.

Alterungsschutzmittel, die insbesondere mit Ozon reagieren und dieses abfangen, werden auch als "Ozonschutzmittel" oder "Antiozonant" bezeichnet.

Der Erfindung liegt die Aufgabe zugrunde, eine neuartige Verbindung bereitzustellen, die insbesondere als Alterungsschutzmittel in Fahrzeugreifen oder anderen technischen Gummiartikeln verwendet werden kann, und zwar mit einem geringeren Gefahrenpotential bei hinreichender Löslichkeit in der jeweiligen Matrix, beispielsweise und insbesondere im Polymer. Hierdurch soll unter Verringerung der Gesundheitsschädlichkeit ein weiterhin optimaler oder sogar verbesserter Schutz vor Sauerstoff und Ozon gewährleistet werden sowie die Tendenz zum Ausblühen (engl. "Blooming") verhindert werden.

Gelöst wird die Aufgabe durch die erfindungsgemäße Verbindung gemäß Anspruch 1, die erfindungsgemäße Kautschukmischung enthaltend die Verbindung sowie den erfindungsgemäßen Fahrzeugreifen, der die erfindungsgemäße Kautschukmischung in wenigstens einem Bauteil aufweist. Ferner wird die Aufgabe durch die Verfahren zur Herstellung der Verbindung sowie die Verwendung der Verbindung als Alterungsschutzmittel und/oder Antioxidationsmittel und/oder Antiozonant gelöst.

Die Verbindung gemäß Anspruch 1 kann ferner als Farbstoff verwendet werden.

Die Verbindung gemäß Anspruch 1 weist die Formel I) auf:

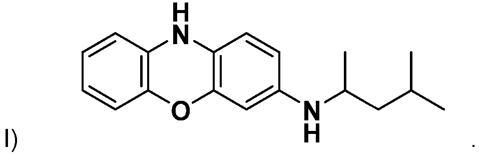

Es handelt sich bei der Verbindung gemäß Formel I) somit um N-(4-methylpentan-2-yl)-1 OH-phenoxazin-3-am ine.

Die Verbindung gemäß Formel I) weist gegenüber den bekannten Alterungsschutzmitteln, wie insbesondere 6PPD, eine erhöhte Reaktivität gegenüber Sauerstoff, Ozon oder Radikalen auf, wodurch mit der Verbindung gemäß Formel I) eine verbesserte Schutzwirkung, insbesondere in Fahrzeugreifen und anderen technischen Gummiartikeln, aber auch in Ölen und Schmierstoffen, erzielt wird.

Die Verbindung gemäß Formel I) ist zudem ein Phenoxazin-Derivat und als solches weniger toxisch als bekannte Diamin-Alterungsschutzmittel, wie 6PPD oder IPPD.

Die erfindungsgemäße Verbindung zeigt in Kautschukmischungen eine gegenüber dem 6PPD verbesserte Alterungsschutzwirkung und ist somit als Ersatz von 6PPD geeignet, dessen Abbauprodukte extrem toxisch für den Silberlachs und damit wohl auch für andere aquatische Organismen sind.

Die erfindungsgemäße Verbindung weist zudem überraschenderweise eine sehr gute Löslichkeit in Kautschukmischungen, insbesondere für Fahrzeugreifen und andere technische Gummiartikel, was auf die 1,3-Dimethylbutyl-Gruppe am Stickstoffatom zurückzuführen sein könnte.

Die Erfindung soll aber prinzipiell nicht an einen bestimmten Wirkmechanismus oder eine bestimmte Erklärung gebunden sein.

Von der Erfindung sind sämtliche vorteilhafte Ausgestaltungen, die sich unter anderem in den Patentansprüchen widerspiegeln, umfasst. Insbesondere sind von der Erfindung auch Ausgestaltungen umfasst, die sich durch Kombination unterschiedlicher Merkmale unterschiedlicher Abstufungen bei der Bevorzugung dieser Merkmale ergeben, sodass auch eine Kombination eines ersten als "bevorzugt" bezeichneten Merkmals oder im Rahmen einer vorteilhaften Ausführungsform beschriebenen Merkmals mit einem weiteren als z. B. "besonders bevorzugt" bezeichneten Merkmal von der Erfindung umfasst ist.

Die erfindungsgemäße Verbindung gemäß Formel I) ist besonders als Alterungsschutzmittel und/oder Ozonschutzmittel in Fahrzeugreifen und/oder anderen technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen geeignet.

Die erfindungsgemäße Verbindung gemäß Formel I) ist besonders zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon geeignet.

Zu Verwendung der Verbindung gemäß Formel I) in den genannten Artikeln oder Stoffen wird diese in einer Zusammensetzung verwendet und in dieser eingemischt verwendet.

Bei Fahrzeugreifen oder anderen technischen Gummiartikeln ist dies insbesondere eine Kautschukmischung.

Ein weiterer Gegenstand der Erfindung ist die Verwendung der erfindungsgemäßen Verbindung gemäß Formel I) in Ölen, Schmierstoffen, wie insbesondere Treib- oder Betriebsstoffen für Motoren. Insbesondere kann die erfindungsgemäße Verbindung somit in Motoren verwendet werden.

Ein weiterer Gegenstand der Erfindung ist die Verwendung der erfindungsgemäßen Verbindung gemäß Formel I) als Farbstoff in Fasern und/oder Polymeren und/oder Papier und/oder in (Streich-)Farben und Lacken.

Ein weiterer Gegenstand der Erfindung ist wie oben ausgeführt eine Kautschukmischung.

Die erfindungsgemäße Kautschukmischung enthält die Verbindung gemäß Formel I). Bei der erfindungsgemäßen Kautschukmischung kann es sich prinzipiell um jede Kautschukmischung handeln, insbesondere in der die neuartige erfindungsgemäße Verbindung gemäß Formel I) als Alterungsschutzmittel und/oder Ozonschutzmittel bei geringerer Toxizität wirkt.

Die erfindungsgemäße Kautschukmischung enthält wenigstens einen Kautschuk.

Bevorzugt enthält die erfindungsgemäße Kautschukmischung 0,1 bis 10 phr, besonders bevorzugt 0,1 bis 6 phr, ganz besonders bevorzugt 1 bis 5 phr, der Verbindung gemäß Formel I).

Die in dieser Schrift verwendete Angabe phr (parts per hundred parts of rubber by weight) ist die in der Kautschukindustrie übliche Mengenangabe für Mischungsrezepturen. Die Dosierung der Gewichtsteile der einzelnen Substanzen wird in dieser Schrift auf 100 Gewichtsteile der gesamten Masse aller in der Mischung vorhandenen hochmolekularen (M_{w} größer als 20.000 g/mol) Kautschuke bezogen.

Gemäß vorteilhafter Ausführungsformen der Erfindung enthält die erfindungsgemäße Kautschukmischung wenigstens einen Dienkautschuk.

Die Kautschukmischung kann somit einen Dienkautschuk oder ein Gemisch von zwei oder mehreren verschiedenen Dienkautschuken enthalten.

Als Dienkautschuke werden Kautschuke bezeichnet, die durch Polymerisation oder Copolymerisation von Dienen und/oder Cycloalkenen entstehen und somit entweder in der Hauptkette oder in den Seitengruppen C=C-Doppelbindungen aufweisen.

Der Dienkautschuk ist dabei bevorzugt ausgewählt aus der Gruppe bestehend aus natürlichem Polyisopren (NR), synthetischem Polyisopren (IR), epoxidiertem Polyisopren (ENR), Butadien-Kautschuk (BR), Butadien-Isopren-Kautschuk, lösungspolymerisiertem Styrol-Butadien-Kautschuk (SSBR), emulsionspolymerisiertem Styrol-Butadien-Kautschuk (ESBR), Styrol-Isopren-Kautschuk, Flüssigkautschuken mit einem Molekulargewicht M_{w} von größer als 20000 g/mol, Halobutyl-Kautschuk, Polynorbornen, Isopren-Isobutylen-Copolymer, Ethylen-Propylen-Dien-Kautschuk, Nitril-Kautschuk, Chloropren-Kautschuk, Acrylat-Kautschuk, Fluor-Kautschuk, Silikon-Kautschuk, Polysulfid-Kautschuk, Epichlorhydrin-Kautschuk, Styrol-Isopren-Butadien-Terpolymer, hydriertem Acrylnitrilbutadien-Kautschuk und hydriertem Styrol-Butadien-Kautschuk.

Insbesondere Nitrilkautschuk, hydrierter Acrylnitrilbutadienkautschuk, Chloroprenkautschuk, Butylkautschuk, Halobutylkautschuk und/oder Ethylen-Propylen-Dien-Kautschuk kommen bei der Herstellung von technischen Gummiartikeln, wie Gurte, Riemen und Schläuche, und/oder Schuhsohlen zum Einsatz. Dabei finden die dem Fachmann für diese Kautschuke bekannten - im Hinblick auf Füllstoffe, Weichmacher, Vulkanisationssysteme und Zuschlagstoffe besonderen - Mischungszusammensetzungen bevorzugte Anwendung.

Bei dem natürlichen und/oder synthetischen Polyisopren sämtlicher Ausführungsformen kann es sich sowohl um cis-1,4-Polyisopren als auch um 3,4-Polyisopren handeln. Bevorzugt ist allerdings die Verwendung von cis-1,4-Polyisoprenen mit einem cis-1,4 Anteil > 90 Gew.-%. Zum einen kann solch ein Polyisopren durch stereospezifische Polymerisation in Lösung mit Ziegler-Natta-Katalysatoren oder unter Verwendung von fein verteilten Lithiumalkylen erhalten werden. Zum anderen handelt es sich bei Naturkautschuk (NR) um ein solches cis-1,4 Polyisopren, bei welchem der cis-1,4-Anteil im Naturkautschuk größer 99 Gew.-% ist.

Ferner ist auch ein Gemisch eines oder mehrerer natürlicher Polyisoprene mit einem oder mehreren synthetischen Polyisopren(en) denkbar.

Im Rahmen der vorliegenden Erfindung ist unter dem Begriff "Naturkautschuk" natürlich vorkommender Kautschuk zu verstehen, der von Hevea Gummibäumen und "Nicht-Hevea" Quellen gewonnen werden kann. Nicht-Hevea Quellen sind beispielsweise Guayule Sträucher und Löwenzahn wie beispielsweise TKS (Taraxacum kok-saghyz; Russischer Löwenzahn).

Falls in der erfindungsgemäßen Kautschukmischung Butadien-Kautschuk (= BR, Polybutadien) enthalten ist, kann es sich um alle dem Fachmann bekannten Typen handeln. Darunter fallen u.a. die sogenannten high-cis- und low-cis-Typen, wobei Polybutadien mit einem cis-Anteil größer oder gleich 90 Gew.-% als high-cis-Typ und Polybutadien mit einem cis-Anteil kleiner als 90 Gew.-% als low-cis-Typ bezeichnet wird. Ein low-cis-Polybutadien ist z.B. Li-BR (Lithium-katalysierter Butadien-Kautschuk) mit einem cis-Anteil von 20 bis 50 Gew.-%. Mit einem high-cis BR werden besonders gute Eigenschaften sowie eine niedrige Hysterese der Kautschukmischung erzielt.

Das oder die eingesetzte(n) Polybutadiene kann/können mit Modifizierungen und Funktionalisierungen endgruppenmodifiziert und/oder entlang der Polymerketten funktionalisiert sein. Bei der Modifizierung kann es sich um solche mit Hydroxy-Gruppen und/oder Ethoxy-Gruppen und/oder Epoxy-Gruppen und/oder Siloxan-Gruppen und/oder Amino-Gruppen und/oder Aminosiloxan und/oder Carboxy-Gruppen und/oder Phthalocyanin-Gruppen und/oder Silan-Sulfid-Gruppen handeln. Es kommen aber auch weitere, dem Fachmann bekannte, Modifizierungen, auch als Funktionalisierungen bezeichnet, in Frage. Bestandteil solcher Funktionalisierungen können Metallatome sein.

Für den Fall, dass wenigstens ein Styrol-Butadien-Kautschuk (Styrol-Butadien-Copolymer) in der Kautschukmischung enthalten ist, kann es sich sowohl um lösungspolymerisierten Styrol-Butadien-Kautschuk (SSBR) als auch um emulsionspolymerisierten Styrol-Butadien-Kautschuk (ESBR) handeln, wobei auch ein Gemisch aus wenigstens einem SSBR und wenigstens einem ESBR eingesetzt werden kann. Die Begriffe "Styrol-Butadien-Kautschuk" und "Styrol-Butadien-Copolymer" werden im Rahmen der vorliegenden Erfindung synonym verwendet.

Das eingesetzte Styrol-Butadien-Copolymer kann mit den oben beim Polybutadien genannten Modifizierungen und Funktionalisierungen endgruppenmodifiziert und/oder entlang der Polymerketten funktionalisiert sein.

Vorzugsweise ist der wenigstens eine Dienkautschuk ausgewählt aus der Gruppe bestehend aus natürlichem Polyisopren (NR, Naturkautschuk), synthetischem Polyisopren (IR), Butadien-Kautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

Gemäß einer besonders bevorzugten Ausführungsform der Erfindung ist der wenigstens eine Dienkautschuk ausgewählt aus der Gruppe bestehend aus natürlichem Polyisopren (NR), synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer besonders vorteilhaften Ausführungsform der Erfindung enthält die Kautschukmischung wenigstens ein natürliches Polyisopren (NR) und zwar bevorzugt in Mengen von 50 bis 100 phr, und gemäß einer besonders vorteilhaften Ausführungsform der Erfindung 80 bis 100 phr, ganz besonders bevorzugt 95 bis 100 phr, wiederum bevorzugt 100 phr. Eine derartige Kautschukmischung zeigt insbesondere optimierte Reißeigenschaften und Abriebeigenschaften bei einer guten Verarbeitbarkeit und Reversionsstabilität.

Für den Fall, dass die Kautschukmischung weniger als 100 phr NR enthält, enthält sie bevorzugt als weiteren Kautschuk wenigstens einen Dienkautschuk, der ausgewählt ist aus der Gruppe bestehend aus synthetischem Polyisopren (IR), Butadien-Kautschuk (BR), lösungspolymerisiertem Styrol-Butadien-Kautschuk (SSBR) und emulsionspolymerisiertem Styrol-Butadien-Kautschuk (ESBR).

Gemäß einer weiteren besonders vorteilhaften Ausführungsform der Erfindung enthält die Kautschukmischung wenigstens ein natürliches Polyisopren (NR) und zwar bevorzugt in Mengen von 5 bis 55 phr, und gemäß einer besonders vorteilhaften Ausführungsform der Erfindung 5 bis 25 phr, ganz besonders bevorzugt 5 bis 20 phr. Eine derartige Kautschukmischung zeigt insbesondere eine gute Verarbeitbarkeit und Reversionsstabilität sowie optimierte Reißeigenschaften und ein optimales Rollwiderstandsverhalten.

Gemäß einer weiteren besonders vorteilhaften Ausführungsform der Erfindung enthält die Kautschukmischung wenigstens ein Polybutadien (BR, Butadienkautschuk) und zwar bevorzugt in Mengen von 10 bis 80 phr, besonders bevorzugt 10 bis 50 phr, und gemäß einer besonders vorteilhaften Ausführungsform der Erfindung 15 bis 40 phr. Hiermit werden besonders gute Reiß- und Abriebeigenschaften der erfindungsgemäßen Kautschukmischung und ein optimales Bremsverhalten erzielt.

Gemäß einer weiteren besonders vorteilhaften Ausführungsform der Erfindung enthält die Kautschukmischung wenigstens einen lösungspolymerisierten Styrol-Butadien-Kautschuk (SSBR) und zwar bevorzugt in Mengen von 10 bis 80 phr, besonders bevorzugt 30 bis 80 phr, und gemäß einer besonders vorteilhaften Ausführungsform der Erfindung 50 bis 70 phr. Hiermit werden besonders gute Rollwiderstandseigenschaften der erfindungsgemäßen Kautschukmischung erzielt. Gemäß besonders vorteilhaften Ausführungsformen der Erfindung wird SSBR in Kombination mit wenigstens einem weiteren Kautschuk eingesetzt, um ein optimales und ausbalanciertes Eigenschaftsprofil zu erzielen.

Bevorzugt enthält die Kautschukmischung wenigstens einen Füllstoff, bevorzugt in Mengen von 30 bis 500 phr, besonders bevorzugt 50 bis 400 phr, wiederum bevorzugt 80 bis 300 phr.

Gemäß vorteilhafter Ausführungsformen der Erfindung ist der Füllstoff ein verstärkender Füllstoff, der bevorzugt ausgewählt ist aus der Gruppe bestehend aus Rußen und Siliciumdioxid.

Als Ruße kommen alle dem Fachmann bekannten Rußtypen in Frage. Bevorzugt ist der Ruß ausgewählt aus Industrierußen und Pyrolyse-Rußen, wobei Industrieruße weiter bevorzugt sind.

Bevorzugt hat der Ruß eine Jodzahl, gemäß ASTM D 1510, die auch als Jodadsorptionszahl bezeichnet wird, zwischen 30 und 250 g/kg, bevorzugt 30 bis 180 g/kg, besonders bevorzugt 40 bis 180 g/kg, und ganz besonders bevorzugt 40 bis 130 g/kg, und eine DBP-Zahl gemäß ASTM D 2414 von 30 bis 200 ml/100 g, bevorzugt 70 bis 200 ml/100g, besonders bevorzugt 90 bis 200 ml/100g.

Die DBP-Zahl gemäß ASTM D 2414 bestimmt das spezifische Absorptionsvolumen eines Rußes oder eines hellen Füllstoffes mittels Dibutylphthalat.

Die Verwendung eines solchen Rußtyps in der Kautschukmischung, insbesondere für Fahrzeugreifen, gewährleistet einen bestmöglichen Kompromiss aus Abriebwiderstand und Wärmeaufbau, der wiederum den ökologisch relevanten Rollwiderstand beeinflusst.

Besonders geeignet und bevorzugt ist dabei ein Ruß mit einer Jodadsorptionszahl zwischen 80 und 110 g/kg und einer DBP-Zahl von 100 bis 130 ml/100g, wie insbesondere Rußes des Types N339.

Das Siliciumdioxid ist bevorzugt amorphes Siliciumdioxid, beispielsweise gefällte Kieselsäure, die auch als gefälltes Siliciumdioxid bezeichnet wird. Alternativ kann aber beispielsweise auch pyrogenes Siliciumdioxid eingesetzt werden. Besonders bevorzugt ist es allerdings, wenn eine fein verteilte, gefällte Kieselsäure verwendet wird, die eine Stickstoff-Oberfläche (BET-Oberfläche) (gemäß DIN ISO 9277 und DIN 66132) von 35 bis 400 m²/g, bevorzugt von 35 bis 350 m²/g, besonders bevorzugt von 85 bis 320 m²/g und ganz besonders bevorzugt von 120 bis 235 m²/g, und eine CTAB-Oberfläche (gemäß ASTM D 3765) von 30 bis 400 m²/g, bevorzugt von 30 bis 330 m²/g, besonders bevorzugt von 80 bis 300 m²/g und ganz besonders bevorzugt von 115 bis 200 m²/g, aufweist. Derartige Kieselsäuren führen z. B. in Kautschukmischungen für Reifenlaufstreifen zu besonders guten physikalischen Eigenschaften der Vulkanisate. Außerdem können sich dabei Vorteile in der Mischungsverarbeitung durch eine Verringerung der Mischzeit bei gleichbleibenden Produkteigenschaften ergeben, die zu einer verbesserten Produktivität führen. Als Kieselsäuren können somit z. B. sowohl jene des Typs Ultrasil^{®} VN3 (Handelsname) der Firma Evonik als auch hoch dispergierbare Kieselsäuren, so genannte HD-Kieselsäuren (z. B. Zeosil^{®} 1165 MP der Firma Solvay), zum Einsatz kommen.

Gemäß besonders vorteilhafter Ausführungsformen der Erfindung enthält die Kautschukmischung wenigstens eine Kieselsäure als Füllstoff, bevorzugt in Mengen von 30 bis 500 phr, besonders bevorzugt 50 bis 400 phr, wiederum bevorzugt 80 bis 300 phr.

In diesen Mengen ist Kieselsäure insbesondere als alleiniger oder als Hauptfüllstoff (mehr als 50 Gew.-% bezogen auf die Gesamtfüllstoffmenge) enthalten.

Gemäß weiterer vorteilhafter Ausführungsformen der Erfindung enthält die Kautschukmischung wenigstens eine Kieselsäure als weiteren Füllstoff, und zwar bevorzugt in Mengen von 5 bis 100 phr, besonders bevorzugt 5 bis 80 phr, wiederum bevorzugt 10 bis 60 phr.

In diesen Mengen ist Kieselsäure insbesondere als weiterer Füllstoff zusätzlich zu einem anderen Hauptfüllstoff, wie insbesondere einem Ruß, enthalten.

Die Begriffe "Kieselsäure" und "Silika" werden im Rahmen der vorliegenden Erfindung synonym verwendet.

Gemäß besonders vorteilhafter Ausführungsformen der Erfindung enthält die erfindungsgemäße Kautschukmischung 0,1 bis 60 phr, bevorzugt 3 bis 40 phr, besonders bevorzugt 5 bis 30 phr, ganz besonders bevorzugt 5 bis 15 phr, wenigstens eines Rußes. In diesen Mengen ist Ruß insbesondere als weiterer Füllstoff zusätzlich zu einem Hauptfüllstoff, wie insbesondere Kieselsäure, enthalten.

Gemäß weiterer vorteilhafter Ausführungsformen der Erfindung enthält die erfindungsgemäße Kautschukmischung 30 bis 300 phr, bevorzugt 30 bis 200 phr, besonders bevorzugt 40 bis 100 phr wenigstens eines Rußes. In diesen Mengen ist Ruß als alleiniger oder als Hauptfüllstoff und dabei ggf. in Kombination mit Kieselsäure in den oben genannten geringeren Mengen enthalten.

Gemäß einer besonders vorteilhaften Ausführungsform der Erfindung enthält die Kautschukmischung 5 bis 60 phr, besonders bevorzugt 5 bis 40 phr, wenigstens eines Rußes und 50 bis 300 phr, bevorzugt 80 bis 200 phr wenigstens einer Kieselsäure.

Die Kautschukmischung kann zudem weitere Füllstoffe enthalten, die verstärkend wirken oder nicht verstärkend wirken.

Zu den weiteren (nicht verstärkenden) Füllstoffen zählen im Rahmen der vorliegenden Erfindung Alumosilicate, Kaolin, Kreide, Stärke, Magnesiumoxid, Titandioxid oder Kautschukgele sowie Fasern (wie zum Beispiel Aramidfasern, Glasfasern, Carbonfasern, Cellulosefasern).

Weitere ggf. verstärkende Füllstoffe sind z.B. Kohlenstoffnanoröhrchen (carbon nanotubes (CNT) inklusive diskreter CNTs, sogenannte hollow carbon fibers (HCF) und modifizierte CNT enthaltend eine oder mehrere funktionelle Gruppen, wie Hydroxy-, Carboxy und Carbonyl-Gruppen), Graphit und Graphene und sogenannte "carbon-silica dual-phase filler".

Zinkoxid gehört im Rahmen der vorliegenden Erfindung nicht zu den Füllstoffen.

Des Weiteren kann die Kautschukmischung übliche Zusatzstoffe in üblichen Gewichtsteilen enthalten, die bei deren Herstellung bevorzugt in wenigstens einer Grundmischstufe zugegeben werden. Zu diesen Zusatzstoffen zählen
a) im Stand der Technik bekannte Alterungsschutzmittel,
   wie z. B. p-Phenylendiamine, wie
   N-Phenyl-N'-(1,3-dimethylbutyl)-p-phenylendiamin (6PPD),
   N,N'-Diphenyl-p-phenylendiamin (DPPD),
   N-(1-phenylethyl)-N'-phenyl-p-phenylendiamin (SPPD),
   N,N'-Ditolyl-p-phenylendiamin (DTPD),
   N-(1,4-dimethylpentyl)-N'-phenyl-p-phenylendiamin (7PPD),
   N-Isopropyl-N'-phenyl-p-phenylendiamin (IPPD),
   oder Dihydrochinoline, wie 2,2,4-Trimethyl-1,2-dihydrochinolin (TMQ),
b) Aktivatoren, wie z. B. Zinkoxid und Fettsäuren (z. B. Stearinsäure) und/oder sonstige Aktivatoren, wie Zinkkomplexe wie z.B. Zinkethylhexanoat,
c) Aktivatoren und/oder Agenzien für die Anbindung von Füllstoffen, insbesondere Ruß oder Kieselsäure, wie beispielsweise S-(3-Aminopropyl)Thioschwefelsäure und/oder deren Metallsalze (Anbindung an Ruß) sowie Silan-Kupplungsagenzien (Anbindung an Siliciumdioxid, insbesondere Kieselsäure),
d) Ozonschutzwachse,
e) Harze, insbesondere Klebharze,
f) Mastikationshilfsmittel, wie z. B. 2,2'-Dibenzamidodiphenyldisulfid (DBD) und
g) Prozesshilfsmittel, wie insbesondere Fettsäureester und Metallseifen, wie z.B. Zinkseifen und/oder Calciumseifen
h) Weichmacher, wie insbesondere wie aromatische, naphthenische oder paraffinische Mineralölweichmacher, wie z.B. MES (mild extraction solvate) oder RAE (Residual Aromatic Extract) oder TDAE (treated distillate aromatic extract), oder Rubber-to-Liquid-Öle (RTL) oder Biomass-to-Liquid-Öle (BTL) bevorzugt mit einem Gehalt an polycyclischen Aromaten von weniger als 3 Gew.-% gemäß Methode IP 346 oder Triglyceride, wie z. B. Rapsöl, oder Faktisse oder Kohlenwasserstoffharze oder Flüssig-Polymere, deren mittleres Molekulargewicht (Bestimmung per GPC = gel permeation chromatography, in Anlehnung an BS ISO 11344:2004) zwischen 500 und 20000 g/mol liegt.

Bei der Verwendung von Mineralöl ist dieses bevorzugt ausgewählt aus der Gruppe, bestehend aus DAE (Destillated Aromatic Extracts), RAE (Residual Aromatic Extract), TDAE (Treated Destillated Aromatic Extracts), MES (Mild Extracted Solvents) und naphthenischen Ölen.

Gemäß besonders vorteilhafter Ausführungsformen enthält die erfindungsgemäße Kautschukmischung neben der erfindungsgemäßen Verbindung gemäß Formel I) keine Alterungsschutzmittel aus der Gruppe der p-Phenylendiamine, insbesondere solche unter obiger Auflistung a). Insbesondere enthält die erfindungsgemäße Kautschukmischung gemäß einer besonders bevorzugten Ausführungsform 0 bis 0,1 phr, insbesondere 0 phr, an weiteren Alterungsschutzmitteln auf Basis von p-Phenylendiaminen, die ausgewählt sind aus der Gruppe enthaltend, bevorzugt bestehend aus, N-Phenyl-N'-(1,3-dimethylbutyl)-p-phenylendiamin (6PPD), N-(1-phenylethyl)-N'-phenyl-p-phenylendiamin (SPPD), N,N'-Diphenyl-p-phenylendiamin (DPPD), N,N'-Ditolyl-p-phenylendiamin (DTPD), N-Isopropyl-N'-phenyl-p-phenylendiamin (IPPD), N-(1,4-dimethylpentyl)-N'-phenyl-p-phenylendiamin (7PPD).

Mit den bevorzugt sehr geringen Mengen von 0 bis 0,1 phr bzw. besonders bevorzugt 0 phr an den genannten p-Phenylendiaminen und der erfindungsgemäß enthaltenen Verbindung gemäß Formel I) ist es möglich eine vergleichbare Schutzwirkung bei geringerer Toxizität zu erzielen. Hierbei ersetzt die erfindungsgemäße Verbindung gemäß Formel I) die genannten im Stand der Technik bekannten p-Phenylendiamine.

Gemäß weiterer vorteilhafter Ausführungsformen der Erfindung ist noch wenigstens ein weiteres der genannten p-Phenylendiamin-Alterungsschutzmittel enthalten, sodass die erfindungsgemäße Verbindung die im Stand der Technik bekannten p-Phenylendiamine nur teilweise ersetzt. Hierdurch wird der erfindungsgemäße Vorteil auch erzielt, nur nicht in optimalem Ausmaß.

Alterungsschutzmittel auf Basis von Dihydrochinolin, wie TMQ, sind gemäß vorteilhafter Ausführungsformen neben der erfindungsgemäßen Verbindung gemäß Formel I) in der Kautschukmischung enthalten. Die Menge an enthaltenen Dihydrochinolinen, wie insbesondere TMQ, beträgt bevorzugt 0,1 bis 3, insbesondere 0,5 bis 1,5 phr.

Ozonschutzwachse (obige Gruppe d) werden separat betrachtet und sind gemäß bevorzugter Ausführungsformen der Erfindung in der Kautschukmischung enthalten, unabhängig davon ob zusätzliche Alterungsschutzmittel a) enthalten sind.

Bei den Silan-Kupplungsagenzien kann es sich um alle dem Fachmann bekannten Typen handeln.

Ferner können ein oder mehrere verschiedene Silan-Kupplungsagenzien in Kombination miteinander eingesetzt werden. Die Kautschukmischung kann somit ein Gemisch verschiedener Silane enthalten.

Die Silan-Kupplungsagenzien reagieren mit den oberflächlichen Silanolgruppen des Siliciumdioxids, insbesondere der Kieselsäure, oder anderen polaren Gruppen während des Mischens des Kautschuks bzw. der Kautschukmischung (in situ) oder bereits vor der Zugabe des Füllstoffes zum Kautschuk im Sinne einer Vorbehandlung (Vormodifizierung).

Aus dem Stand der Technik bekannten Kupplungsagenzien sind bifunktionelle Organosilane, die am Siliciumatom mindestens eine Alkoxy-, Cycloalkoxy- oder Phenoxygruppe als Abgangsgruppe besitzen und die als andere Funktionalität eine Gruppe aufweisen, die gegebenenfalls nach Spaltung eine chemische Reaktion mit den Doppelbindungen des Polymers eingehen kann. Bei der letztgenannten Gruppe kann es sich z. B. um die folgenden chemischen Gruppen handeln: -SCN, -SH, -NH₂ oder -Sₓ- (mit x = 2 bis 8).

So können als Silan-Kupplungsagenzien z. B. 3-Mercaptopropyltriethoxysilan, 3-Thiocyanato-propyltrimethoxysilan oder 3,3'-Bis(triethoxysilylpropyl)polysulfide mit 2 bis 8 Schwefelatomen, wie z. B. 3,3'-Bis(triethoxysilylpropyl)tetrasulfid (TESPT), das entsprechende Disulfid (TESPD) oder auch Gemische aus den Sulfiden mit 1 bis 8 Schwefelatomen mit unterschiedlichen Gehalten an den verschiedenen Sulfiden, verwendet werden. TESPT kann dabei beispielsweise auch als Gemisch mit Industrieruß (Handelsname X50S^{®} der Firma Evonik) zugesetzt werden.

Auch geblockte Mercaptosilane, wie sie z. B. aus der WO 99/09036 bekannt sind, können als Silan-Kupplungsagens eingesetzt werden. Auch Silane, wie sie in der WO 2008/083241 A1, der WO 2008/083242 A1, der WO 2008/083243 A1 und der WO 2008/083244 A1 beschrieben sind, können eingesetzt werden. Verwendbar sind z. B. Silane, die unter dem Namen NXT in verschiedenen Varianten von der Firma Momentive, USA, wie insbesondere 3-Octanoylthio-1-propyltriethoxysilan, oder solche, die unter dem Namen VP Si 363^{®} von der Firma Evonik Industries vertrieben werden.

Der Mengenanteil der Gesamtmenge an weiteren Zusatzstoffen beträgt bevorzugt 3 bis 150 phr, besonders bevorzugt 3 bis 100 phr und ganz besonders bevorzugt 5 bis 80 phr.

Im Gesamtmengenanteil der weiteren Zusatzstoffe kann Zinkoxid (ZnO) in den oben genannten Mengen enthalten sein.

Hierbei kann es sich um alle dem Fachmann bekannten Typen an Zinkoxid handeln, wie z.B. ZnO-Granulat oder -Pulver. Das herkömmlicherweise verwendete Zinkoxid weist in der Regel eine BET-Oberfläche von weniger als 10 m²/g auf. Es kann aber auch ein Zinkoxid mit einer BET-Oberfläche von 10 bis 100 m²/g, wie z.B. so genannte "nano-Zinkoxide", verwendet werden.

Die erfindungsgemäße Kautschukmischung wird bevorzugt vulkanisiert verwendet, insbesondere in Fahrzeugreifen oder anderen vulkanisierten technischen Gummiartikeln.

Die Begriffe "vulkanisiert" und "vernetzt" werden im Rahmen der vorliegenden Erfindung synonym verwendet.

Die Vulkanisation der erfindungsgemäßen Kautschukmischung wird bevorzugt in Anwesenheit von Schwefel und/oder Schwefelspendern mit Hilfe von Vulkanisationsbeschleunigern durchgeführt, wobei einige Vulkanisationsbeschleuniger zugleich als Schwefelspender wirken können. Dabei ist der Beschleuniger ausgewählt aus der Gruppe bestehend aus Thiazolbeschleunigern, Mercaptobeschleunigern, Sulfenamidbeschleunigern, Thiocarbamatbeschleunigern, Thiurambeschleunigern, Thiophosphatbeschleunigern, Thioharnstoffbeschleunigern, Xanthogenat-Beschleunigern und Guanidin-Beschleunigern. Bevorzugt ist die Verwendung eines Sulfenamidbeschleunigers, der ausgewählt ist aus der Gruppe bestehend aus N-Cyclohexyl-2-benzothiazolsufenamid (CBS), N,N-Dicyclohexylbenzothiazol-2-sulfenamid (DCBS), Benzothiazyl-2-sulfenmorpholid (MBS), N-tert-Butyl-2-benzothiazylsulfenamid (TBBS) und Guanidin-Beschleunigern wie Diphenylguanidin (DPG).

Als schwefelspendende Substanz können dabei alle dem Fachmann bekannten schwefelspendenden Substanzen verwendet werden.

Außerdem können in der Kautschukmischung Vulkanisationsverzögerer vorhanden sein.

Die Herstellung der Kautschukmischung erfolgt bevorzugt ansonsten nach dem in der Kautschukindustrie üblichen Verfahren, bei dem zunächst in ein oder mehreren Mischstufen eine Grundmischung mit allen Bestandteilen außer dem Vulkanisationssystem (z. B. Schwefel und vulkanisationsbeeinflussende Substanzen) hergestellt wird. Durch Zugabe des Vulkanisationssystems in einer letzten Mischstufe wird die Fertigmischung erzeugt.

Die Fertigmischung wird z.B. durch einen Extrusionsvorgang oder Kalandrieren weiterverarbeitet und in die entsprechende Form gebracht.

Die erfindungsgemäße Kautschukmischung ist besonders für die Verwendung in Fahrzeugreifen, insbesondere Fahrzeugluftreifen geeignet. Hierbei ist die Anwendung in allen Reifenbauteilen prinzipiell denkbar, insbesondere in einem äußeren Bauteil, insbesondere und bevorzugt im Hornprofil, Laufstreifen und/oder der Seitenwand Laufstreifen. Im Falle eines Laufstreifens mit Cap/Base-Konstruktion wird die erfindungsgemäße Kautschukmischung bevorzugt wenigstens in der Cap verwendet.

Zur Verwendung in Fahrzeugreifen wird die Mischung als Fertigmischung vor der Vulkanisation in die entsprechende Form, bevorzugt eines äußeren Bauteils, gebracht und bei der Herstellung des Fahrzeugreifenrohlings wie bekannt aufgebracht.

Die Herstellung der erfindungsgemäßen Kautschukmischung zur Verwendung als sonstige Body-Mischung in Fahrzeugreifen erfolgt wie bereits beschrieben. Der Unterschied liegt in der Formgebung nach dem Extrusionsvorgang bzw. dem Kalandrieren der Mischung. Die so erhaltenen Formen der noch unvulkanisierten Kautschukmischung für eine oder mehrere unterschiedliche Body-Mischungen dienen dann dem Aufbau eines Reifenrohlings.

Als Body-Mischung werden hierbei die Kautschukmischungen für die inneren Bauteile eines Reifen bezeichnet, wie im Wesentlichen Squeegee, Innenseele (Innenschicht), Kernprofil, Gürtel, Schulter, Gürtelprofil, Karkasse, Wulstverstärker, Wulstprofil, Hornprofil und Bandage.

Der noch unvulkanisierte Reifenrohling wird anschließend vulkanisiert.

Zur Verwendung der erfindungsgemäßen Kautschukmischung in Riemen und Gurten, insbesondere in Fördergurten, wird die extrudierte noch unvulkanisierte Mischung in die entsprechende Form gebracht und dabei oder nachher häufig mit Festigkeitsträgern, z.B. synthetische Fasern oder Stahlcorde, versehen. Zumeist ergibt sich so ein mehrlagiger Aufbau, bestehend aus einer und/oder mehrerer Lagen Kautschukmischung, einer und/oder mehrerer Lagen gleicher und/oder verschiedener Festigkeitsträger und einer und/oder mehreren weiteren Lagen dergleichen und/oder einer anderen Kautschukmischung.

Ein weiterer Gegenstand der vorliegenden Erfindung ist ein Fahrzeugreifen, der die erfindungsgemäße Kautschukmischung enthaltend die erfindungsgemäße Verbindung in wenigstens einem Bauteil aufweist.

Der vulkanisierte Fahrzeugreifen weist wenigstens in einem Bauteil ein Vulkanisat wenigstens einer erfindungsgemäßen Kautschukmischung auf. Dem Fachmann ist bekannt, dass die meisten Substanzen, wie z. B. die enthaltenen Kautschuke entweder bereits nach dem Mischen oder erst nach der Vulkanisation in chemisch veränderter Form vorliegen oder vorliegen können.

Unter Fahrzeugreifen werden im Rahmen der vorliegenden Erfindung Fahrzeugluftreifen und Vollgummireifen, inklusive Reifen für Industrie- und Baustellenfahrzeuge, LKW-, PKW- sowie Zweiradreifen verstanden.

Bevorzugt weist der erfindungsgemäße Fahrzeugreifen die erfindungsgemäße Kautschukmischung in wenigstens einem äußeren Bauteil auf, wobei das äußere Bauteil bevorzugt ein Laufstreifen, eine Seitenwand und/oder ein Hornprofil ist.

Der erfindungsgemäße Fahrzeugreifen kann die erfindungsgemäße Kautschukmischung enthaltend die erfindungsgemäße Verbindung gemäß Formel I) somit auch in mehreren Bauteilen in ggf. angepasster Zusammensetzung aufweisen.

Ein weiterer Gegenstand der vorliegenden Erfindung ist ein Verfahren zur Herstellung der Verbindung gemäß Formel I), wobei das Verfahren wenigstens die folgenden Verfahrensschritte umfasst:
a1) Bereitstellung der Substanz gemäß Formel A1) und A2): 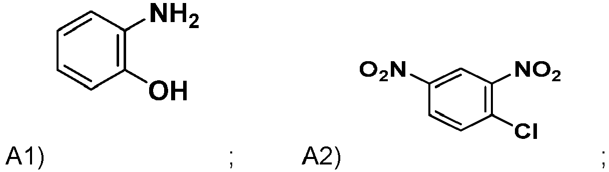
b1) Bereitstellung eines Acetates, insbesondere
   Natriumacetat (NaOAc) oder Kaliumacetat (KOAc), bevorzugt Natriumacetat (NaOAc);
c1) Umsetzung der Substanzen gemäß Schritt a1) in Gegenwart der Substanz gemäß Schritt b1) zu der Verbindung gemäß Formel C1): 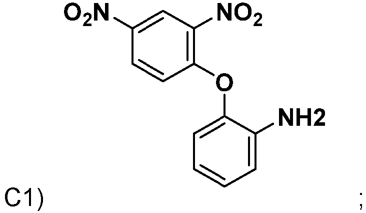
d1) Bereitstellung einer Base, insbesondere Natriummethanolat (NaOMe);
e1) Umsetzung der Verbindung gemäß Formel C1) in Gegenwart der Substanz gemäß Schritt d1) zu der Verbindung gemäß Formel E1): 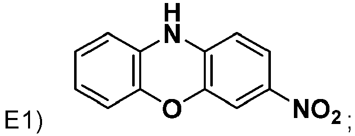
f1) Bereitstellung von Methylisobutylketon (MIBK) sowie Wasserstoff;
g1) Umsetzung der Verbindung gemäß Formel E1) mit den Substanzen aus Schritt f1) zu der Verbindung gemäß Formel I):

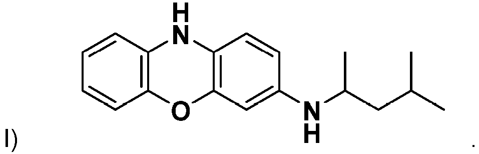

Bevorzugt wird bei der Umsetzung mit Wasserstoff ein geeigneter Katalysator, im Rahmen der vorliegenden Erfindung als "Hydrierungskatalysator" bezeichnet, verwendet.

Bevorzugt ist der Hydrierungskatalysator ein Edelmetallkatalysator, wie insbesondere Palladium (Pd) oder Platin (Pt). Bevorzugt wird das Edelmetall auf Kohle (C) eingesetzt, wie Palladium auf Kohle (Pd/C).

Ferner können auch andere bekannte Katalysatoren, wie Raney-Nickel oder Kupferchromit verwendet werden.

Besonders bevorzugt erfolgt damit die Umsetzung in Schritt g1) mit Wasserstoff. unter Verwendung eines Hydrierungskatalysators.

Bevorzugt erfolgt die Umsetzung in Schritt g1) bei einer Temperatur von 80 bis 150 °C, insbesondere zum Beispiel 120 °C.

Bevorzugt wird Wasserstoff mit einem Druck von 35 bis 70 bar, besonders bevorzugt 45 bis 55 bar, insbesondere zum Beispiel 50 bar, aufgedrückt und bevorzugt anschließend für 1 bis 20 Stunden, bevorzugt 8 bis 13 Stunden, insbesondere zum Beispiel 10 Stunden, gerührt.

Bevorzugt erfolgt die Umsetzung mit Wasserstoff in Schritt g1) in einem für den bevorzugt vergleichsweise hohen Druck geeigneten Behälter, wie insbesondere in einem Autoklav oder in einem anderem Druckreaktor.

Besonders bevorzugt erfolgt die Umsetzung in Schritt g1) mit Wasserstoff unter Verwendung eines Hydrierungskatalysators und bei einer Temperatur von 80 bis 150 °C und wobei Wasserstoff mit einem Druck von 35 bis 70 bar, besonders bevorzugt 45 bis 55 bar, insbesondere zum Beispiel 50 bar aufgedrückt wird und die Reaktion in einem Autoklav oder in einem anderen Druckreaktor stattfindet.

Das Lösungsmittel in Schritt g1) kann entweder das gleichzeitig als Reaktant dienende MIBK oder ein inertes Lösungsmittel, wie Toluol oder Xylol sein. Im letzteren Fall wird MIBK nur in stöchiometrischen Mengen als Reaktant eingesetzt. Bevorzugt wird jedoch MIBK gleichzeitig als Lösungsmittel verwendet. Hierdurch kann auf eine zusätzliche Substanz, wie Toluol oder Xylol, verzichtet werden. Zudem kann nicht umgesetztes MIBK im Anschluss an die Reaktion wiedergewonnen und erneut verwendet werden.

Bevorzugt erfolgt im Anschluss an Schritt g1) eine Aufreinigung, wie beispielsweise säulenchromatographisch, beispielsweise an Kieselgel.

Im Folgenden soll die Erfindung anhand von Ausführungsbeispielen näher erläutert werden.

Die Verbindung gemäß Formel I) wurde auf folgende Weise gemäß einer ersten Syntheseroute hergestellt:
Zunächst wurde wie oben ausgeführt die Verbindung E1) gemäß den Schritten a1) bis e1) hergestellt, wie auch in der US 3413291 beschrieben.

Anschließend erfolgte hieraus die Umsetzung gemäß Schritt g1), wie folgt beschrieben:

### Synthese von N-(4-methylpentan-2-yl)-10H-phenoxazine-3-amin:

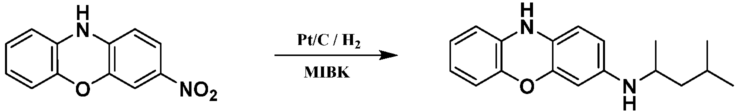

In einen Edelstahlautoklaven, der mit einem Tefloninliner bestückt wurde, wurden 1.00 g (4.38 mmol, 1 Äq) 3-Nitro-10H-phenoxazin, 0.375 g Platin auf Kohle (5%) (0.4 g auf 4.67 mmol Substrat) und 20.0 mL Methylisobutylketon eingewogen. Anschließend wurde 50 bar Wasserstoff aufgedrückt und bei 120°C für 10 Stunden rühren gelassen. Nach Beendigung der Reaktion wurde der überschüssige Wasserstoff abgeblasen und die Suspension über Celite^{®} filtriert sowie mit Ethanol nachgewaschen. Das Filtrat wurde bis zur Trockne eingeengt und im Vakuum getrocknet. Der Rückstand wurde per LC-MS analysiert. Das Ergebnis ist in Tabelle 1 zusammengefasst.

**Tabelle 1**

| **Substanz** | **Ausbeute [%]** | **Retentionszeit [min]** |
|---|---|---|
| 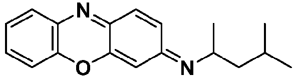 | 37 | 3.79 |
| 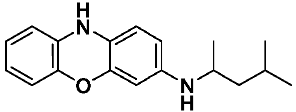 | 38 | 4.78 |

Das erhaltene Substanzgemisch umfassend die Verbindung gemäß Formel I) kann ohne Aufreinigung als Bestandteil einer Kautschukmischung zugegeben werden.

Ein weiterer Aspekt der vorliegenden Erfindung ist ein Gemisch umfassend die Substanzen gemäß Formel I) und Formel II): 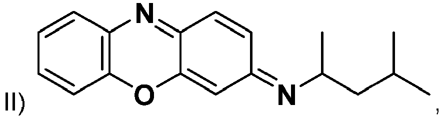 wobei die Substanz gemäß Formel I) bevorzugt mit einem Gewichtsanteil von 30 bis 90 Gew.-% und die Substanz gemäß Formel II) bevorzugt mit einem Gewichtsanteil von 10 bis 50 Gew.-% enthalten ist.

Die Substanz gemäß Formel II) entsteht insbesondere aus der Substanz gemäß Formel I). Ferner kann die Substanz gemäß Formel II) auch wieder zur Substanz gemäß Formel I) regenerieren.

Gemäß einer vorteilhaften Ausführungsform der Erfindung enthält das Gemisch 30 bis 80 Gew.-%, bevorzugt 30 bis 50 Gew.-%, der Substanz gemäß Formel I) und 10 bis 50 Gew.-%, bevorzugt 30 bis 50 Gew.-%, der Substanz gemäß Formel II).

Das Gemisch kann ferner Dimerisierungsprodukte oder oxidierte Derivate, wie insbesondere Phenolate, der Substanzen gemäß Formel I) und/oder II) enthalten.

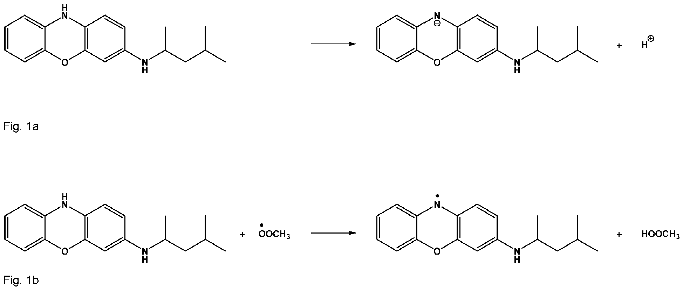

Die erfindungsgemäße Verbindung gemäß Formel I) weist gegenüber 6PPD eine erhöhte Reaktivität, auf. Dies ließ sich beispielsweise anhand der Berechnung von Bindungsdissoziationsenergien (BDE), sowie der freien Aktivierungsenthalpie Δ_{R}G^{≠} bzw. der freien Standardreaktionsenthalpie Δ_{R}G° für die Reaktion mit einem Methylperoxidradikal nachvollziehen. Die Werte sind in Tabelle 2 angegeben. In Fig.1a und 1b sind die Spaltmechanismen dargestellt, auf die sich die Werte beziehen.

**Tabelle 2**

| **Molekül** | **BDE [kJ/mol]** | **Δ_{R}G° [kJ/mol]** | **Δ_{R}G^{≠} [kJ/mol]** |
|---|---|---|---|
| 6PPD | 313 | -25,6 | 19,1 |
| Formel I) | 237 | -63,0 | 0,8 |

Wie Tabelle 2 zeigt, ergeben sich für die erfindungsgemäße Verbindung gemäß Formel I) eine niedrigere Bindungsdissoziationsenergie und niedrigere freie Enthalpien.

### Messung der Oxidations-Induktions-Zeit (OIT, engl. "oxidation induction time")

Die Verbindung gemäß Formel I) wurde durch Messung der Oxidations-Induktions-Zeit unter Laborbedingungen auf ihre potenzielle Schutzwirkung als Alterungsschutzmittel untersucht.

Hierzu wurden die Verbindungen gemäß Formel I) in Form des Substanzgemisches wie in Tabelle 1) wiedergegeben sowie 6-PPD jeweils zusammen mit einem Polymer (flüssiges synthetisches Polyisopren (IR), LIR-50, Fa. Kuraray, Gewichtsmittel der Molekulargewichtsverteilung M_{w} = 54.000 g/mol, Glasübergangstemperatur T_{g} = -63°C) bei konstanter Temperatur (180°C) erhitzt bis Oxidation eintrat (Starttemperatur 35 °C, Aufheizen auf 170°C mit einer Heizrate von 20 K/min (Kelvin pro Minute), Aufheizen auf 180°C mit einer Heizrate von 1 K/min; Spülgas: Stickstoff (N₂) mit einem Volumenstrom von 50 mL/min).

Die Probe wurde 5 Minuten bei 180°C isotherm unter N₂-Atmosphäre gehalten und anschließend wurde auf O₂-Atmosphäre (mit einem Volumenstrom von 50 mL/min) umgestellt.

Die Oxidation wurde über einen Peak mittels DSC (dynamische Differenzkalorimetrie; *engl.* "differential scanning calorimetry") ermittelt.

Gemessen wurde die Zeit in Minuten bis zur Oxidation.

Die Ergebnisse sind im Vergleich zu dem bekannten Alterungsschutzmittel 6PPD in Tabelle 3 zusammengefasst.

**Tabelle 3**

| **Substanz** | **Zeit [min]** |
|---|---|
| 6PPD | 116 ± 10 |
| Gemisch, umfassend Substanz gemäß Formel I) | 104 ± 10 |

Unter Berücksichtigung der Messgenauigkeit von ± (Plusminus) 10 Minuten zeigt sich, dass das Gemisch umfassend die Verbindung gemäß Formel I) eine gegenüber 6PPD vergleichbare Schutzwirkung erzielt. Damit ist die erfindungsgemäße Verbindung gemäß Formel I) umwelt- und gesundheitsfreundlicher als 6-PPD bzw. weitere Vertreter der Substanzklasse, wie eingangs ausgeführt, und zudem ein besseres Alterungsschutzmittel.

Mit der Verbindung gemäß Formel I) kann somit eine vergleichbare Schutzwirkung bei den genannten Anwendungsmöglichkeiten erzielt werden.

Für die Anwendung in einer Kautschukmischung für Fahrzeugreifen wird die erfindungsgemäße Verbindung gemäß Formel I) beispielsweise anstelle der im Stand der Technik bekannten Alterungsschutzmittel, wie 6PPD, 7PPD oder IPPD usw., auf dem Fachmann bekannte Weise in einer der Mischstufen bei der Herstellung der Kautschukmischung zugegeben.

Die Verbindung gemäß Formel I) wurde hiernach in verschiedenen Mengen in eine beispielhafte erfindungsgemäße Kautschukmischung, wie in Tabelle 4 gezeigt, eingemischt. Dabei wurde das oben erhaltene Substanzgemisch (Tabelle 1) verwendet. Die sich ergebenden erfindungsgemäßen Beispiele sind mit E1 und E2 gekennzeichnet.

Als Vergleich dienen Kautschukmischungen enthaltend 6PPD anstelle der Verbindung gemäß Formel I) als Alterungsschutzmittel bei sonst gleicher Zusammensetzung, wobei zwischen V1 und E1 sowie V2 und E2 jeweils ein molgleicher Austausch stattfand. Die Mengen in Tabelle 4 sind in der Einheit phr angegeben. Ferner ist eine Referenz (Ref.) ohne Alterungsschutzmittel angegeben. Bei sämtlichen Mischungen beträgt die Summe der Mengen an Alterungsschutzmittel (6PPD oder Formel I) und Weichmacheröl MES 10 phr.

**Tabelle 4**

| **Bestandteil** | **V1** | **E1** | **V2** | **E2** | **Ref.** |
|---|---|---|---|---|---|
| NR | 100 | 100 | 100 | 100 | 100 |
| Ruß N 339 | 50 | 50 | 50 | 50 | 50 |
| MES | 9 | 8,96 | 7 | 6,88 | 10 |
| 6PPD | 1 | - | 3 | - | - |
| Verbindung gemäß Formel I) | - | 1,04 | - | 3,12 | - |
| ZnO | 3 | 3 | 3 | 3 | 3 |
| Stearinsäure | 2 | 2 | 2 | 2 | 2 |
| TBBS | 1,2 | 1,2 | 1,2 | 1,2 | 1,2 |
| Schwefel | 1,2 | 1,2 | 1,2 | 1,2 | 1,2 |

Bei den erfindungsgemäßen Beispielen zeigt sich keine signifikante Verschlechterung oder sogar eine Verbesserung in der Alterungsschutzwirkung gegenüber Kautschukmischungen mit 6PPD.

## Patentansprüche

1. Verbindung gemäß Formel I)

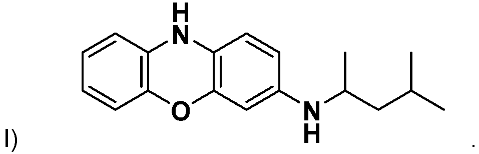

2. Kautschukmischung enthaltend die Verbindung gemäß Formel I) nach Anspruch 1.

3. Kautschukmischung nach Anspruch 2, **dadurch gekennzeichnet, dass** sie wenigstens einen Dienkautschuk enthält.

4. Kautschukmischung nach Anspruch 3, **dadurch gekennzeichnet, dass** sie wenigstens einen Dienkautschuk enthält, der ausgewählt ist aus der Gruppe bestehend aus natürlichem Polyisopren (NR), synthetischem Polyisopren (IR), Butadienkautschuk (BR), lösungspolymerisiertem Styrol-Butadien-Kautschuk (SSBR), emulsionspolymerisiertem Styrol-Butadien-Kautschuk (ESBR), Butylkautschuk (IIR) und Halobutylkautschuk.

5. Fahrzeugreifen, der die Kautschukmischung nach einem der Ansprüche 2 bis 4 in wenigstens einem Bauteil aufweist.

6. Fahrzeugreifen nach Anspruch 5, **dadurch gekennzeichnet, dass** er wenigstens eine Kautschukmischung nach einem der Ansprüche 2 bis 4 in wenigstens einem äußeren Bauteil aufweist, wobei das äußere Bauteil bevorzugt ein Laufstreifen, eine Seitenwand und/oder ein Hornprofil ist.

7. Verfahren zur Herstellung der Verbindung gemäß Formel I) nach Anspruch 1 umfassend wenigstens die folgenden Verfahrensschritte:
a1) Bereitstellung der Substanz gemäß Formel A1) und A2): 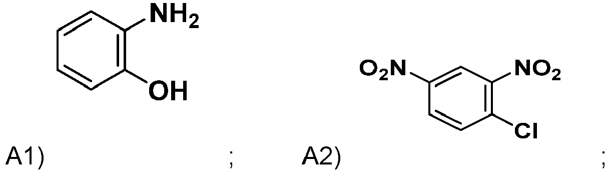
b1) Bereitstellung eines Acetates, insbesondere Natriumacetat (NaOAc) oder Kaliumacetat (KOAc), bevorzugt Natriumacetat (NaOAc);
c1) Umsetzung der Substanzen gemäß Schritt a1) in Gegenwart der Substanz gemäß Schritt b1) zu der Verbindung gemäß Formel C1): 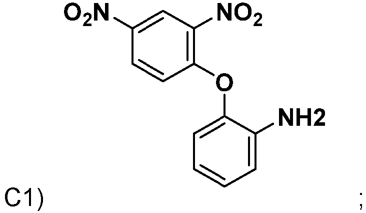
d1) Bereitstellung einer Base, insbesondere Natriummethanolat (NaOMe);
e1) Umsetzung der Verbindung gemäß Formel C1) in Gegenwart der Substanz gemäß Schritt d1) zu der Verbindung gemäß Formel E1): 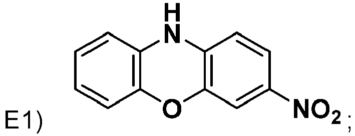
f1) Bereitstellung von Methylisobutylketon (MIBK) sowie Wasserstoff;
g1) Umsetzung der Verbindung gemäß Formel E1) mit den Substanzen aus Schritt f1) zu der Verbindung gemäß Formel I):

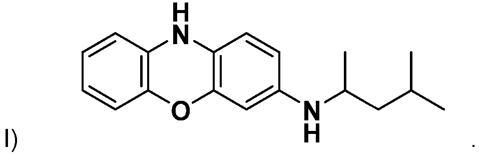

8. Verfahren nach Anspruch 7, wobei die Umsetzung in Schritt g1) mit Wasserstoff unter Verwendung eines Hydrierungskatalysators und/oder bei einer Temperatur von 80 bis 150 °C erfolgt und/oder Wasserstoff mit einem Druck von 35 bis 70 bar, besonders bevorzugt 45 bis 55 bar, insbesondere zum Beispiel 50 bar, aufgedrückt wird und die Reaktion in einem Autoklav oder in einem anderen Druckreaktor stattfindet.

9. Verwendung der Verbindung gemäß Formel I) nach Anspruch 1 als Alterungsschutzmittel und/oder Ozonschutzmittel insbesondere in Fahrzeugreifen und/oder anderen technischen Gummiartikeln, wie insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon, und/oder Ölen und/oder Schmierstoffen.

10. Verwendung der Verbindung gemäß Formel I) nach Anspruch 1 zur Herstellung eines Gummiartikels, insbesondere einer Luftfeder, eines Balgs, Förderbands, Gurtes, Riemens, Schlauchs, Gummibands, Profils, einer Dichtung, einer Membran, taktilen Sensoren für medizinische Anwendungen oder Roboteranwendungen, oder einer Schuhsohle oder Teilen davon.

11. Verwendung der Verbindung gemäß Formel I) nach Anspruch 1 in Ölen, Schmierstoffen, wie insbesondere Treib- oder Betriebsstoffen für Motoren.

12. Verwendung der Verbindung gemäß Formel I) nach Anspruch 1 als Farbstoff in Fasern und/oder Polymeren und/oder Papier und/oder in (Streich-) Farben und Lacken.

## Claims

1. Compound of formula I)

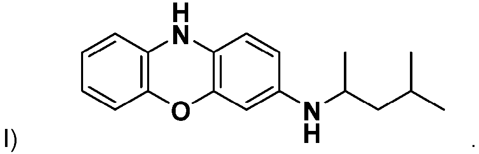

2. Rubber mixture containing the compound of formula I) according to Claim 1.

3. Rubber mixture according to Claim 2, **characterized in that** it contains at least one diene rubber.

4. Rubber mixture according to Claim 3, **characterized in that** it contains at least one diene rubber selected from the group consisting of natural polyisoprene (NR), synthetic polyisoprene (IR), butadiene rubber (BR), solution-polymerized styrene-butadiene rubber (SSBR), emulsion-polymerized styrene-butadiene rubber (ESBR), butyl rubber (IIR) and halobutyl rubber.

5. Vehicle tyre comprising the rubber mixture according to any of Claims 2 to 4 in at least one component.

6. Vehicle tyre according to Claim 5, **characterized in that** it comprises at least one rubber mixture according to any of Claims 2 to 4 in at least one outer component, wherein the outer component is preferably a tread, a sidewall and/or a flange profile.

7. Process for producing the compound of formula I) according to Claim 1 comprising at least the process steps of:
a1) providing the substances of formula A1) and A2): 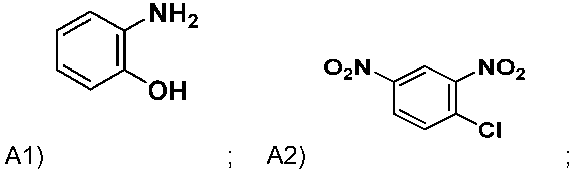
b1) providing an acetate, especially
sodium acetate (NaOAc) or potassium acetate (KOAc), preferably sodium acetate (NaOAc);
c1) reacting the substances of step a1) in the presence of the substance of step b1) to afford the compound of formula C1): 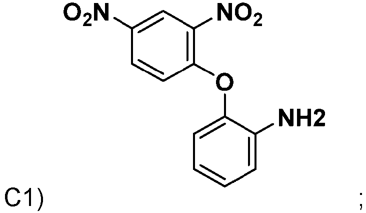
d1) providing a base, especially sodium methoxide (NaOMe);
e1) reacting the compound of formula C1) in the presence of the substance of step d1) to afford the compound of formula E1): 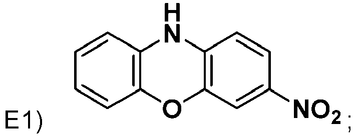
f1) providing methyl isobutyl ketone (MIBK) and hydrogen;
g1) reacting the compound of formula E1) with the substances from step f1) to afford the compound of formula I):

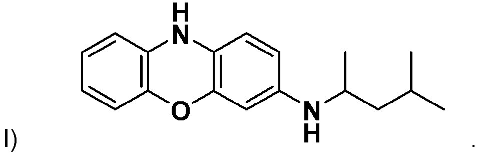

8. Process according to Claim 7, wherein the reaction in step g1) is carried out with hydrogen using a hydrogenation catalyst and/or at a temperature of 80°C to 150°C and/or the reaction mixture is subjected to hydrogen at a pressure of 35 to 70 bar, particularly preferably 45 to 55 bar, especially for example 50 bar, and the reaction is carried out in an autoclave or in another pressure reactor.

9. Use of the compound of formula I) according to Claim 1 as an aging stabilizer and/or antiozonant in particular in vehicle tyres and/or other technical rubber articles, such as especially an air spring, a bellows, a conveyor belt, a strap, a drive belt, a hose, a rubber band, a profile, a seal, a membrane, tactile sensors for medical applications or robotics applications or a shoe sole or parts thereof and/or oils and/or lubricants.

10. Use of the compound of formula I) according to Claim 1 for producing a rubber article, in particular an air spring, a bellows, a conveyor belt, a strap, a drive belt, a hose, a rubber band, a profile, a seal, a membrane, tactile sensors for medical applications or robotics applications or a shoe sole or parts thereof.

11. Use of the compound of formula I) according to Claim 1 in oils, lubricants, such as especially fuels or fluids for engines.

12. Use of the compound of formula I) according to Claim 1 as a dye in fibres and/or polymers and/or paper and/or in (decorating) paints and coatings.

## Revendications

1. Composé selon la formule I)

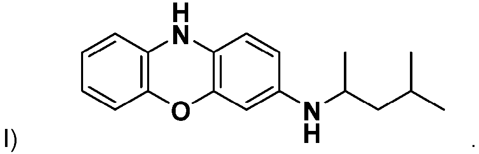

2. Mélange de caoutchouc contenant le composé selon la formule I) selon la revendication 1.

3. Mélange de caoutchouc selon la revendication 2, **caractérisé en ce qu'**il contient au moins un caoutchouc diénique.

4. Mélange de caoutchouc selon la revendication 3, **caractérisé en ce qu'**il contient au moins un caoutchouc diénique, qui est choisi dans le groupe constitué par le polyisoprène naturel (NR), le polyisoprène synthétique (IR), le caoutchouc de butadiène (BR), le caoutchouc de styrène-butadiène polymérisé en solution (SSBR), le caoutchouc de styrène-butadiène polymérisé en émulsion (ESBR), le caoutchouc butyle (IIR) et le caoutchouc halogénobutyle.

5. Pneumatique pour véhicule qui présente le mélange de caoutchouc selon l'une des revendications 2 à 4 dans au moins un composant.

6. Pneumatique pour véhicule selon la revendication 5, **caractérisé en ce qu'**il présente au moins un mélange de caoutchouc selon l'une des revendications 2 à 4 dans au moins un composant externe, le composant externe étant de préférence une bande de roulement, un flanc latéral et/ou un profilé de protection.

7. Procédé de préparation du composé selon la formule I) selon la revendication 1, comprenant au moins les étapes de procédé suivantes :
a1) mise à disposition de la substance selon les formules A1) et A2) : 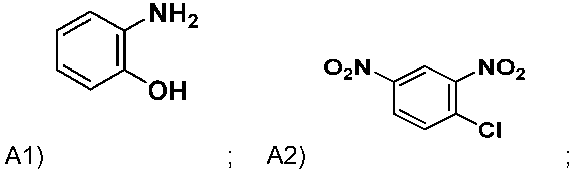
b1) mise à disposition d'un acétate, en particulier
l'acétate de sodium (NaOAc) ou l'acétate de potassium (KOAc), de préférence l'acétate de sodium (NaOAc) ;
c1) transformation des substances selon l'étape a1) en présence de la substance selon l'étape b1) en composé selon la formule C1) : 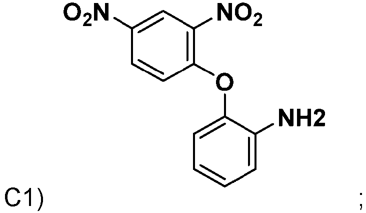
d1) mise à disposition d'une base, en particulier le méthanolate de sodium (NaOMe) ;
e1) transformation du composé selon la formule C1) en présence de la substance selon l'étape d1) en composé selon la formule E1) : 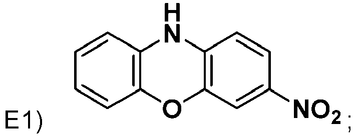
f1) mise à disposition de méthylisobutylcétone (MIBK) et d'hydrogène ;
g1) transformation du composé selon la formule E1) avec les substances de l'étape f1) en composé selon la formule I):

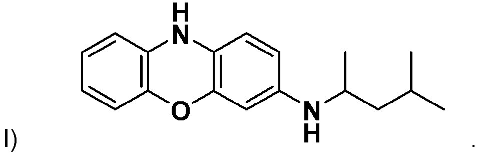

8. Procédé selon la revendication 7, la transformation dans l'étape g1) avec de l'hydrogène ayant lieu avec utilisation d'un catalyseur d'hydrogénation et/ou à une température de 80 à 150°C et/ou l'hydrogène étant comprimé à une pression de 35 à 70 bars, de manière particulièrement préférée de 45 à 55 bars, en particulier par exemple de 50 bars, et la réaction ayant lieu dans une autoclave ou dans un autre réacteur sous pression.

9. Utilisation du composé selon la formule I) selon la revendication 1 comme agent de protection contre le vieillissement et/ou contre l'ozone, en particulier dans des pneumatiques de véhicule et/ou d'autres articles techniques en caoutchouc, tels qu'en particulier un ressort pneumatique, un soufflet, une bande transporteuse, une sangle, une courroie, un tuyau, une bande en caoutchouc, un profilé, un joint, une membrane, des capteurs tactiles pour des applications médicales ou robotiques ou une semelle de chaussure ou des parties de celle-ci et/ou des huiles et/ou des lubrifiants.

10. Utilisation du composé selon la formule I) selon la revendication 1 pour la fabrication d'un article en caoutchouc, en particulier d'un ressort pneumatique, d'un soufflet, d'une bande transporteuse, d'une sangle, d'une courroie, d'un tuyau, d'une bande en caoutchouc, d'un profilé, d'un joint, d'une membrane, de capteurs tactiles pour des applications médicales ou robotiques ou d'une semelle de chaussure ou des parties de celle-ci.

11. Utilisation du composé selon la formule I) selon la revendication 1 dans des huiles, des lubrifiants, tels qu'en particulier des carburants ou consommables pour des moteurs.

12. Utilisation du composé selon la formule I) selon la revendication 1 comme colorant dans des fibres et/ou des polymères et/ou du papier et/ou des peintures et des laques (d'enduction).
